# EUROPÄISCHE PATENTSCHRIFT

(11) **EP 3 174 849 B1**
(45) Veröffentlichungstag und Bekanntmachung des Hinweises auf die Patenterteilung: **13.03.2019**
(21) Anmeldenummer: 15744170.0
(22) Anmeldetag: 27.07.2015
(51) Int. Cl.: C07D 207/28

(54) **HERSTELLUNG VON PYRROLIDINDERIVATEN**
SYNTHESIS OF PYRROLIDINE DERIVATIVES
PRODUCTION DE DÉRIVÉS DE PYRROLIDINE

(30) Priorität: 30.07.2014 DE 102014110782
(43) Veröffentlichungstag der Anmeldung: 07.06.2017
(73) Patentinhaber: Eberhard Karls Universität Tübingen Medizinische Fakultät, 72074 Tübingen (DE)
(72) Erfinder: HIRT, Bernhard, 72070 Tübingen (DE); ZEYHER, Claus, 73614 Schorndorf (DE)
(74) Vertreter: Witte, Weller & Partner Patentanwälte mbB
(86) Internationale Anmeldenummer: PCT/EP2015/067112
(87) Internationale Veröffentlichungsnummer: WO 2016/016167

(56) Entgegenhaltungen:
- EP-A2- 0 156 275
- WO-A1-2012/028196
- DATABASE REGISTRY [Online] CHEMICAL ABSTRACTS SERVICE, COLUMBUS, OHIO, US; 20. April 1990 (1990-04-20), XP002744825, gefunden im STN Database accession no. 126646-24-0
- DATABASE REGISTRY [Online] CHEMICAL ABSTRACTS SERVICE, COLUMBUS, OHIO, US; 1. November 2010 (2010-11-01), XP002744826, gefunden im stn Database accession no. 1249956-07-7

## Beschreibung

Die vorliegende Erfindung betrifft ein Verfahren zur Herstellung eines Pyrrolidinderivates.

Pyrrolidin ist ein ringförmiges, aliphatisches, sekundäres Amin mit fünf Ringgliedern. Es ist ein Heterozyklus. Pyrrolidin hat die Summenformel C₄H₉N und die CAS-Nummer 123-75-1. Derivate des Pyrrolidins sind Abkömmlinge hiervon, die durch chemische Modifizierung von Pyrrolidin hergestellt werden können. Derivate von Pyrrolidins sind im Stand der Technik umfassend beschrieben.

Ein wichtiges Pyrrolidinderivat ist das sogenannte Glucoprotamin. Glucoprotamin ist eine aus mehreren Komponenten bestehende Substanz. Die wichtigsten Komponenten und Hauptwirksubstanzen von Glucoprotamin sind das (2S)-Pyrrolidin-5-oxo-carbonsäureamid-N-3-(dodecylamino)propyl und das (2S)-Pyrrolidin-5-oxo-carbonsäureamid-N-3-(tetradecylamino)propyl.

Glucoprotamin wird aufgrund seiner antimikrobiellen Wirksamkeit als Flächendesinfektionsmittel, vorwiegend in Krankenhäusern und Kliniken, eingesetzt.

Glucoprotamin entsteht als Umsetzungsprodukt der linearen L-Glutaminsäure bzw. deren Esterderivate und Cocospropylen-1,3-diamin. Neben der Kopplung der Glutaminsäure mit dem Fettamingemisch kommt es bei der Synthesereaktion unter Wasserabspaltung zu einer Zyklisierung und Ausbildung eines für die Wirkweise essentiellen Pyrrolidin-Ringes. Neben den beiden genannten Hauptwirksubstanzen entstehen weitere lang- und kurzkettige Nebenprodukte. Es folgt eine Wasserabscheidung und Aufreinigung des Gemisches zur Gewinnung der Wirksubstanzen.

Das derzeit angewendete Herstellungsverfahren von Glucoprotamin ist in der DE 3 410 956 beschrieben.

Das bekannte Herstellungsverfahren weist eine Reihe von Nachteilen auf. So erfordert die bekannte Herstellung zur Bildung des Ringschlusses unter Wasserbildung eine hohe Reaktionstemperatur von bis zu 175°C. Dabei ist die langkettige Alkylgruppe einer Oxidations- und Zersetzungsgefahr ausgesetzt. Der Einsatz weiterer Derivate, wie ungesättigten Fettsäuren, ist aufgrund der Oxidationsprozesse und Nebenreaktionen nicht möglich. Zur Wasserabscheidung ist ein geeigneter Wasserabscheider und ein hohes Vakuum nötig, weshalb bisher z.B. als Lösungs- und Schleppmittel i-Amylalkohol eingesetzt wird. Dieses Mittel ist jedoch gesundheitsschädlich und muss im Rahmen einer Aufreinigung wieder entfernt werden. Die hohen Reaktionstemperaturen sind nicht nur chemisch gesehen von Nachteil sondern auch zunehmend ökologisch und ökonomisch unrentabel. Ein weiterer Nachteil des bekannten Herstellungsprozesses besteht darin, dass sich im Bereich des Pyrrolidinringes keine spezifisch planbaren Modifikationen einbringen lassen.

Vor diesem Hintergrund ist es die Aufgabe der vorliegenden Erfindung, ein neues Verfahren zur Herstellung von Pyrrolidinderivaten, wie beispielsweise dem Glucoprotamin, bereitzustellen, mit dem die vorstehend beschriebenen Nachteile vermieden oder zumindest reduziert werden.

Diese Aufgabe wird durch ein Verfahren zur Herstellung eines Pyrrolidinderivates gelöst, das folgende Schritte aufweist:
1) Inkubation der Reaktionspartner
   a) 5-Oxo-Pyrrolidin-2-carbonsäure-Derivat mit der Formel I
      a) 5-Oxo-Pyrrolidin-2-carbonsäure-Derivat mit der Formel I 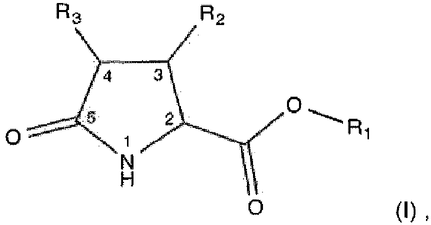 in der
      R₁ ein linearer Alkylrest mit C₁-C₆ ist;
      R₂ und R₃ jeweils unabhängig voneinander ausgewählt sind aus der Gruppe bestehend aus:
         H, XH, 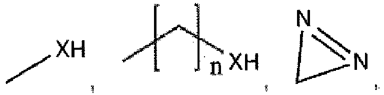 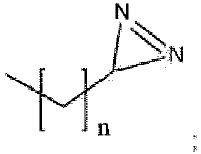 mit jeweils:
         n = 0 - 20, und
         X = O oder S, und
   b) einer Verbindung ausgewählt aus der Gruppe bestehend aus:
      N-substituiertes Diamin mit der Formel II 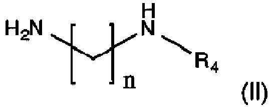 in der R₄ ein linearer Alkyl- oder Acylrest mit C₂ bis C₂₂ ist, und n = 1 bis 6 ist, und
      N-substituiertes Monoamin mit der Formel III 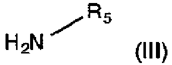 in der R₅ ein linearer Alkyl- oder Acylrest mit C₂ bis C₂₂ ist, und
      N-substituiertes Fettamid mit der Formel IV 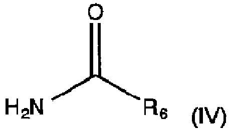 in der R₆ ein linearer Alkylrest mit C₂ bis C₂₄ ist,
         unter geeigneten Reaktionsbedingungen, und
2) Isolierung des erhaltenen Pyrrolidinderivates.

Wie die Erfinder feststellen konnten, lässt sich ausgehend von dem 5-Oxo-pyrrolidin-2-carbonsäure-Derivat und N-substituierten Mono- und/oder Diaminen sowie Fettamiden auf vorteilhafte Art und Weise ein Pyrrolidinderivat herstellen.

Das erfindungsgemäße Verfahren weist gegenüber dem bekannten Herstellungsverfahren eine Vielzahl von Vorteilen auf. So gelingt die erfindungsgemäße Herstellung bereits bei niedrigen Temperaturen, beispielsweise bei ca. 60°C, und bei nur leicht reduziertem Druck, bei beispielsweise ca. 330 mbar. Auch ist die Herstellungsdauer äußerst kurz und liegt bei ca. 60 min. Die Herstellung ist somit schneller, schonender und energieeffizienter als bei dem herkömmlichen Verfahren.

Ferner erfolgt die erfindungsgemäße Herstellung mit weniger Nebenreaktionen. Die Reinheit des Reaktionsproduktes wird dadurch erhöht.

Bei dem erfindungsgemäßen Verfahren entsteht kein Wasser während der Synthesereaktion, das bei der anschließenden Aufreinigung durch einen Wasserabscheider aufwendig getrennt werden müsste.

Das erfindungsgemäße schonende Herstellungsverfahren ermöglicht es, auch vulnerable C₂-₂₂-Alkyl-Derivate z.B. mit ungesättigten Fettsäuren für die Synthese zu verwenden. Weitere Wirkstoffderivate mit anderem Wirkspektrum können generiert und so der Anwendungsbereich der Pyrrolidinderivate erweitert werden.

Das erfindungsgemäße Herstellungsverfahren ermöglicht es außerdem, spezifische Modifikationen der Ausgangssubstanzen durchzuführen, durch die die Wirkstoffkinetik, die Wirkstoffdynamik und die Wirkstoffleistung modulierbar werden.

Eine ggf. chemisch aufwendige Kopplung von Funktionsgruppen an die Ausgangssubstanzen kann vorab in einem eigenen Prozess erfolgen, so dass auch hier der vulnerable Synthesepartner der C₂₋₂₂-Alkyl-Derivate keiner Oxidations- und Zersetzungsgefahr ausgesetzt wird.

Das erfindungsgemäße Verfahren ermöglicht es außerdem, Funktionsgruppen spezifisch einzuführen, die eine Kopplung des Wirkstoffs z.B. an Oberflächenmaterialien ermöglichen.

Schließlich ist das erfindungsgemäße Verfahren im Vergleich zu dem aus der DE 3 410 956 bekannten Verfahren durch seine geringen Kosten gekennzeichnet. Die Fertigungsprozesse finden bei wesentlich geringeren Temperaturen statt, was eine deutliche Senkung des Herstellungsaufwandes bedeutet.

Erfindungsgemäß wird unter einem "Pyrrolidinderivat" ein Abkömmling des Pyrrolidins verstanden, der sich durch chemische Modifizierung von Pyrrolidin gewinnen lässt. Dabei weist das Derivat nach wie vor den Heterozyklus des Pyrrolidins auf. Der Heterozyklus des Pyrrolidins kann an verschiedenen Positionen funktionelle Gruppen aufweisen, wie vorzugsweise an den Positionen 3 und 4. Ein Beispiel für ein Pyrrolidinderivat ist das Glucoprotamin.

Erfindungsgemäß umfasst ein "Derivat" von 5-Oxo-Pyrrolidin-2-carbonsäure ein Abkömmling hiervon, der sich durch chemische Modifizierung hiervon gewinnen lässt. Die Derivate weisen nach wie vor den Heterozyklus des Pyrrolidins auf, können sich jedoch insbesondere in ihren Alkylresten voneinander unterscheiden. Der Heterozyklus des Pyrrolidins kann an verschiedenen Positionen funktionelle Gruppen aufweisen, wie vorzugsweise an den Positionen 3 und 4. Erfindungsgemäß wird sowohl das S-Enantiomer als auch das R-Enantiomer oder das hieraus gebildete Racemat umfasst. Das "5-Oxo-Pyrrolidin-2-carbonsäure-Derivat" umfasst ferner ein "5-Oxo-Pyrrolidin-2-carbonester-Derivat", das auch als Pyroglutamatesterderivat bezeichnet wird.

Erfindungsgemäß werden unter "geeigneten Reaktionsbedingungen" solche Bedingungen verstanden, die eine chemische Umsetzung der Reaktionspartner (a) und (b) zu einem Pyrrolidinderivat ermöglichen. Diese können von einem Fachmann ohne weiteres ermittelt werden und lassen sich je nach spezifischen Reaktionspartnern einstellen.

Nach einer besonderen Ausführungsform des erfindungsgemäßen Verfahrens ist das 5-Oxo-Pyrrolidin-2-carbonsäure-Derivat durch Kopplung zumindest einer funktionellen Gruppe modifiziert.

Diese Maßnahme hat den Vorteil, dass Pyrrolidinderivate mit spezifischen Eigenschaften herstellbar sind. Dabei sind für eine Kopplung besonders die Positionen 3 und/oder 4 des Pyrrolidinringes geeignet.

In einer weiteren Ausführungsform der Erfindung ist die an das 5-Oxo-Pyrrolidin-2-carbonsäure-Derivat gekoppelte funktionelle Gruppe ausgewählt aus der Gruppe bestehend aus: OH-Gruppe, SH-Gruppe und fotoreaktive Gruppe.

Die Anheftung von beispielsweise geschützten Hydroxyl- oder Sulfhydrylgruppen an die 5-Oxo-Pyrrolidin-2-carbonsäure ermöglicht die Kopplung des Pyrrolidinderivates an Oberflächen verschiedener Materialien. Dies erlaubt die Nutzung der Wirksubstanzen als Oberflächenbeschichtung. Durch die Anheftung von fotoreaktiven Gruppen erfolgt eine konditionierte Modifizierung der Wirksubstanz.

In einer weiteren Ausführungsform der Erfindung weist das N-substituierte Diamin und/oder N-substituierte Monoamin und/oder N-substituierte Fettamid an R₄ bzw. R₅ bzw. R₆ einen einfach und/oder mehrfach ungesättigten Alkyl- und/oder Acylrest auf.

Diese Maßnahme hat den Vorteil, dass durch die Wahl der Kettenlänge die Eigenschaften des Pyrrolidinderivates nach Belieben variiert werden können.

Nach einer bevorzugten Ausgestaltung des erfindungsgemäßen Verfahrens ist das 5-Oxo-Pyrrolidin-2-Carbonsäure-Derivat ein 5-Oxo-Pyrrolidin-2-carbonsäuremethylester mit der Formel V: 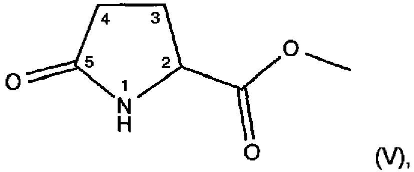 wobei es weiter bevorzugt ist, wenn das N-substituierte Diamin Cocospropylen-1,3-diamin mit der Formel VI ist:

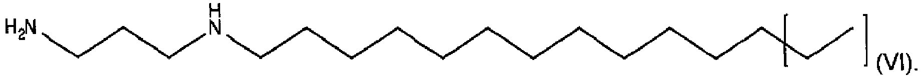

Diese Maßnahme hat den Vorteil, dass solche Reaktionspartner als Ausgangssubstanzen gewählt werden, mit denen eine gezielte Herstellung von Glucoprotamin erfolgt. 5-Oxo-Pyrrolidin-2-carbonsäuremethylester wird auch als L-Pyroglutaminsäuremethylester bezeichnet. Es hat die Summenformel C₆H₉NO₃ und weist ein Molekulargewicht von 143,14 auf. Die CAS-Nummer ist: 4931-66-2. Cocospropylen-1,3-diamin wird auch als N-coco-alkyltrimethylenediamin bezeichnet. Mit einem C12-Rest hat es die Summenformel C₁₅H₃₄NO₂ und ein Molekulargewicht von 242,27, mit einem C14-Rest hat es die Summenformel C₁₇H₃₈N₂ und ein Molekulargewicht von 270,30. Die CAS-Nummer ist: 61791-63-7. Cocospropylen-1,3-diamin ist ein aus Naturstoffen erzeugtes Produkt und unterliegt daher natürlichen Schwankungen bzw. enthält weitere kurz- oder langkettige Alkylreste.

Dabei ist es erfindungsgemäß bevorzugt, wenn das Cocospropylen-1,3-Diamin ca. 70 Mol-% Dodecylpropylendiamin und ca. 30 Mol-% Tetradecylpropylendiamin aufweist.

Nach Erkenntnissen der Erfinder werden durch diese Verhältnismäßigkeit besonders hohe Ausbeuten des Pyrrolidinderivats erzielt, die bei ca. 97-99% der theoretischen Masse liegen.

Nach einer bevorzugten Ausgestaltung des erfindungsgemäßen Verfahrens handelt es sich bei dem hergestellten Pyrrolidinderivat um Glucoprotamin (CAS-Nr. 164907-72-6) mit der Formel VII

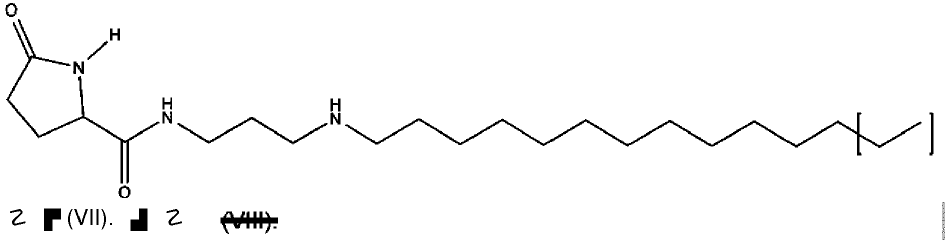

Durch diese Maßnahme wird eines der wichtigsten Flächendesinfektionsmittel aus dem Klinikalltag nunmehr auf vorteilhafte Art und Weise herstellbar. Glucoprotamin wird auch als Amin, N-C12-14-alkylpropylenedi-, L-glutamat bezeichnet. In der Formel symbolisiert die Klammer, dass es sich um länger- oder kürzerkettige Alkylreste handeln kann, die in einer Mischung vorliegen. Mit einem C12-Rest weist Glucoprotamin die Strukturformel C₂₀H₃₉N₃O₂ und das Molekulargewicht 353,55, mit einem C14-Rest die Strukturformel C₂₂H₄₃N₃O₂ und das Molekulargewicht 381,61 auf. Glucoprotamin wird bspw. von der Firma Ecolab Deutschland GmbH, Düsseldorf, vertrieben.

Nach einer bevorzugten Ausgestaltung des erfindungsgemäßen Verfahrens erfolgt die Inkubation der Reaktionspartner bei einer Temperatur von ca. 10°C bis ca. 100°C, vorzugsweise bei ca. 30°C bis ca. 90°C, höchst vorzugsweise bei ca. 60°C.

Diese Maßnahme hat den Vorteil, dass ein deutlich geringerer Energieaufwand zur Herstellung des Pyrrolidinderivates betrieben wird, als in dem aus der DE 3 410 956 bekannten Verfahren. Das Herstellungsverfahren ist damit schonender, energieeffizienter und kostengünstiger. Die niedrigen Temperaturen verhindern außerdem die Zerstörung von langkettigen Alkyl- und Acylgruppen. Gleichzeitig wird jedoch eine optimale Ausbeute sichergestellt.

Dabei ist es erfindungsgemäß bevorzugt, wenn die Reaktionspartner für eine Dauer von ca. 10 Min. bis ca. 100 Min., weiter vorzugsweise für ca. 30 Min. bis ca. 90 Min., und höchst vorzugsweise für ca. 60 Min. inkubiert werden.

Diese Maßnahme hat den Vorteil, dass die Herstellung schnell und damit gleichermaßen schonender als bei dem herkömmlichen Verfahren gelingt. Wie die Erfinder festgestellt haben, sind die bevorzugten Zeiten jedoch ausreichend, um eine zufriedenstellende Ausbeute zu gewährleisten.

Nach einer bevorzugten Ausgestaltung des erfindungsgemäßen Verfahrens erfolgt die Inkubation der Reaktionspartner bei einem Druck von ≤ ca. 1 bar, vorzugsweise von ≤ ca. 800 mbar, weiter vorzugsweise von ≤ ca. 500 mbar, weiter vorzugsweise von ≤ ca. 400 mbar, und höchst vorzugsweise bei ca. 330 mbar.

Wie die Erfinder feststellen konnten, führt die Reduzierung des Druckes unterhalb des Atmosphärendruckes zu einer verbesserten Ausbeute, wobei jedoch die Bereitstellung eines Vakuums, wie im Stand der Technik, nicht erforderlich ist.

Nach einer bevorzugten Ausgestaltung des erfindungsgemäßen Verfahrens ist das molare Verhältnis der Reaktionspartner (a) zu (b) gleich ca. 1 zu ca. 1 bis ca. 1 zu ca. 2.

Diese Maßnahme hat den Vorteil, dass nach Erkenntnissen der Erfinder bei den angegebenen molaren Verhältnissen besonders gute Ausbeuten des Pyrrolidinderivates erzielt werden.

Nach einer bevorzugten Weiterbildung des erfindungsgemäßen Verfahrens werden die in Schritt (1) entstandenen Reaktionsnebenprodukte, wie beispielsweise Methanol, entfernt, beispielsweise durch Destillation.

Diese Maßnahme hat den Vorteil, dass die Hauptwirksubstanzen bzw. das Pyrrolidinderivat in besonders reiner Form erhalten wird.

Ein weiterer Gegenstand der vorliegenden Erfindung betrifft die Verwendung eines 5-Oxo-Pyrrolidin-2-carbonsäure-Derivates mit der Formel I 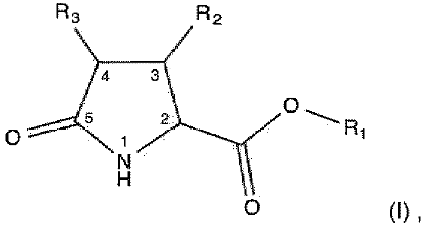 in der
R₁ ein linearer Alkylrest mit C₁-C₆ ist;
R₂ und R₃ jeweils unabhängig voneinander ausgewählt sind aus der Gruppe bestehend aus:
   H, XH, 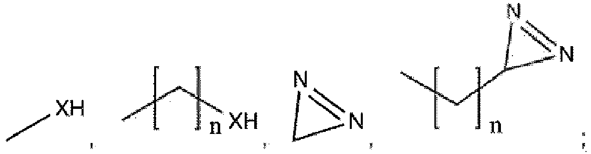 mit jeweils:
   n = 0 - 6, und
   X = O oder S, vorzugsweise von
5-Oxo-Pyrrolidin-2-carbonsäuremethylester mit der Formel V 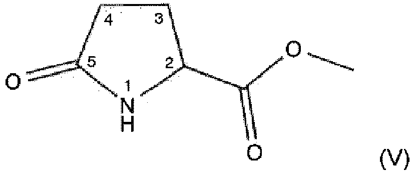 als Ausgangssubstanz zur Herstellung eines Pyrrolidinderivates, vorzugsweise von Glucoprotamin.

Die Erfinder haben erkannt, dass zur Herstellung von Pyrrolidinderivaten, wie dem Glucoprotamin, nicht von der linearen Substanz L-Glutaminsäure bzw. deren Esterderivaten ausgegangen werden muss, sondern vorteilhafterweise von dem bereits zyklisch vorliegenden 5-Oxo-Pyrrolidin-2-carbonsäure-Derivat. Die Synthesereaktion kann so bei deutlich niedrigeren Temperaturen, höherem Druck und in kürzerer Zeit durchgeführt werden. Ferner lässt sich das 5-Oxo-Pyrrolidin-2-carbonsäure-Derivat ohne Weiteres vorab in einem eigenen Prozess modifizieren und somit Pyrrolidinderivate mit gewünschten Eigenschaften herstellen.

Die Merkmale, Eigenschaften, Vorteile und Weiterbildungen des erfindungsgemäßen Verfahrens gelten für die erfindungsgemäße Verwendung entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand von Ausführungsbeispielen näher erläutert, aus denen sich weitere Eigenschaften, Merkmale und Vorteile ergeben. Die Ausführungsbeispiele sind rein illustrativ und schränken die Reichweite der Erfindung nicht ein.

### Ausführungsbeispiele

### 1. Das bekannte Herstellungsverfahren von Glucoprotamin

Das bisherige Herstellungsverfahren des 5-Oxo-Pyrrolidin-2-carbonsäure-Derivates Glucoprotamin ist in der DE 3 410 956 beschrieben und nachfolgend schematisch dargestellt.

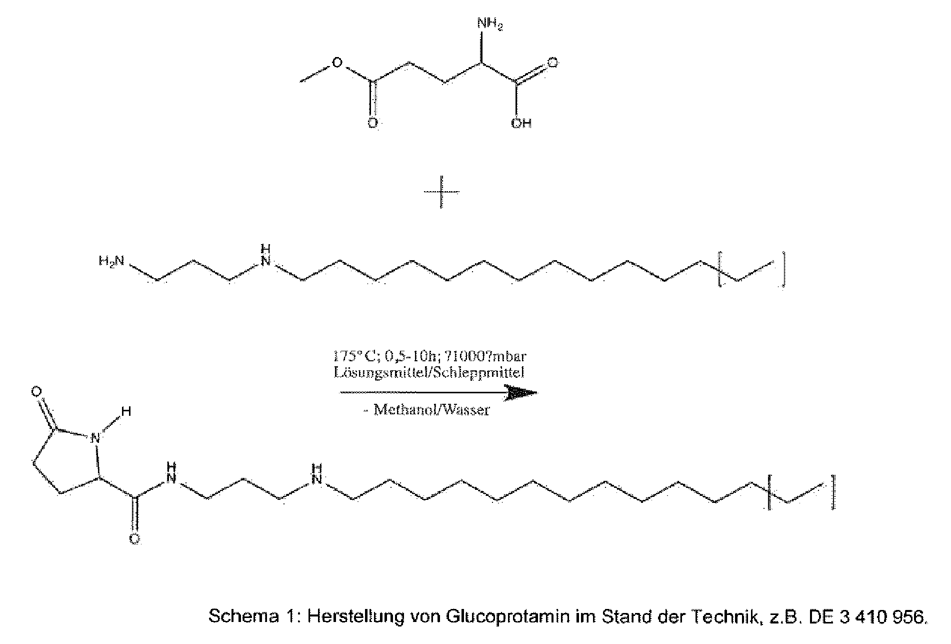

Danach wird lineare L-Glutaminsäure mit Cocospropylen-1,3-diamin unter hohen Temperaturen von bis zu 175°C und langen Reaktionszeiten von bis zu 10 Stunden umgesetzt. Wasser wird mittels eines Wasserabscheiders im Vakuum abgeschieden. Dazu wird als Lösungs- und Schleppmittel i-Amylalkohol eingesetzt.

### 2. Das erfindungsgemäße Herstellungsverfahren

Die Reaktion findet mit den Ausgangssubstanzen
I. 5-Oxo-Pyrrolidin-2-carbonsäure-Derivaten (bevorzugt das S-Enantiomer, aber auch das *R*-Enantiomer oder Racemat) und
II. N-substituierten Monoaminen (2.3) und/oder Diaminen (2.2) und/oder Fettamiden (2.3) statt.

### 2.1 Umsetzung von 5-Oxo-Pyrrolidin-2-carbonsäure-Derivaten mit N-substituierten Monoaminen

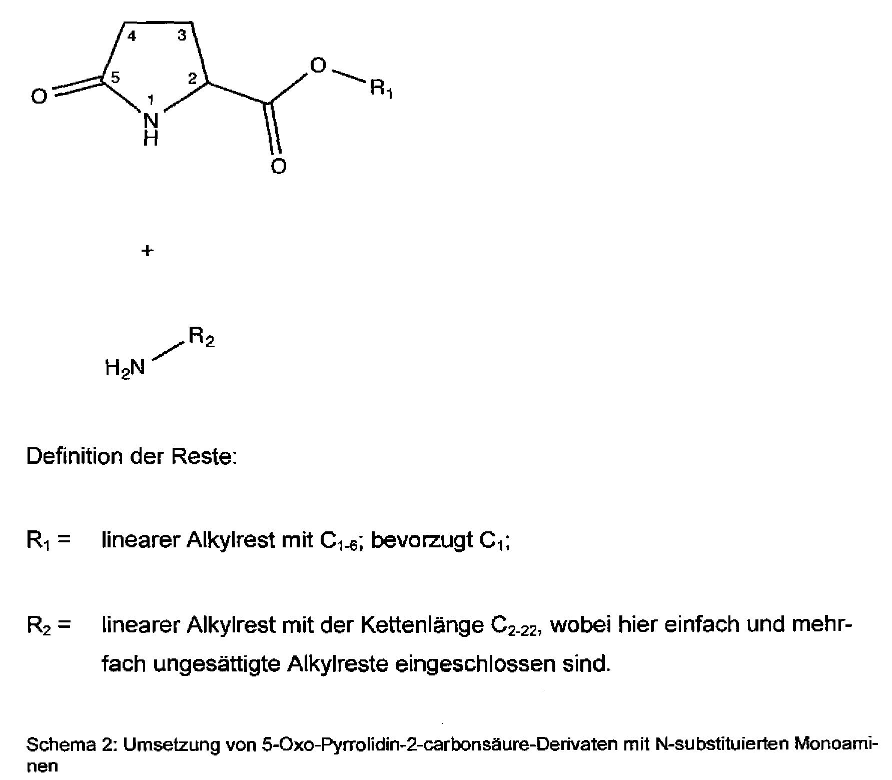

Die Reaktion erfordert lediglich eine bevorzugte Temperatur von 60°C, eine bevorzugte Reaktionsdauer von 60 min. und einen bevorzugten Druck von 300-350 mbar. Methanol wird abdestilliert.

### 2.2 Umsetzung von 5-Oxo-Pyrrolidin-2-carbonsäure-Derivaten mit N-substituierten Diaminen

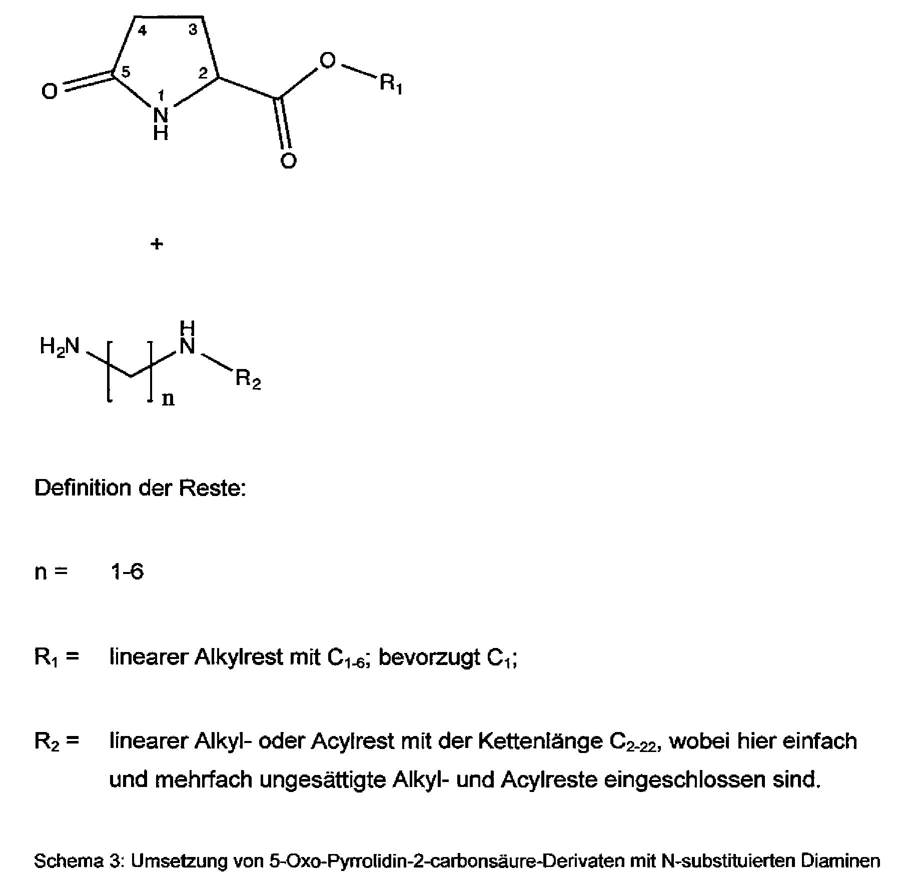

Die Reaktion erfordert lediglich eine bevorzugte Temperatur von 60°C, eine bevorzugte Reaktionsdauer von 60 min. und einen bevorzugten Druck von 300-350 mbar. Methanol wird abdestilliert.

### 2.3 Umsetzung von 5-Oxo-Pyrrolidin-2-carbonsäure-Derivaten mit N-substituierten Fettamiden

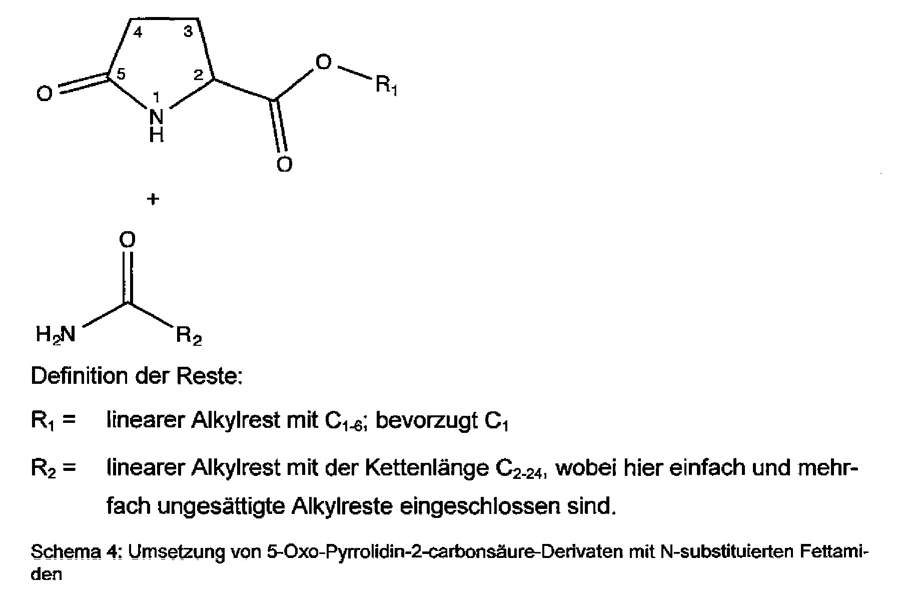

Die Reaktion erfordert lediglich eine bevorzugte Temperatur von 60°C, eine bevorzugte Reaktionsdauer von 60 min. und einen bevorzugten Druck von 300-350 mbar. Methanol wird abdestilliert.

### 3. Erfindungsgemäße Synthese von Glucoprotamin

### 3.1 Allgemeine Beschreibung

Die unter dem Wirkstoff Glucoprotamin subsumierten beiden Wirksubstanzen (2S)-Pyrrolidin-5-oxo-carbonsäureamid-N-3-(dodecylamino)propyl und (2S)-Pyrrolidin-5-oxo-carbonsäureamid-N-3-(tetradecylamino)propyl werden in dem erfindungsgemäßen Verfahren nicht durch Reaktion der linearen Ausgangssubstanz L-Glutaminsäure bzw. deren Esterderivate und dem Fettamingemisch Dodecyl-/Tetradecylpropylendiamin, auch als Cocospropylen-1,3-diamin bezeichnet, erzeugt, sondern durch die Reaktion des bereits zyklisch vorliegenden Ausgangsproduktes 5-Oxo-Pyrrolidin-2(S)-carbonsäuremethylester, das auch als L-Pyroglutaminsäuremethylester bezeichnet wird, mit Cocospropylen-1,3-diamin bei nur ca. 60°C, ca. 60 min. und ca. 300 bis 350 mbar unter Abdestillation von Methanol.

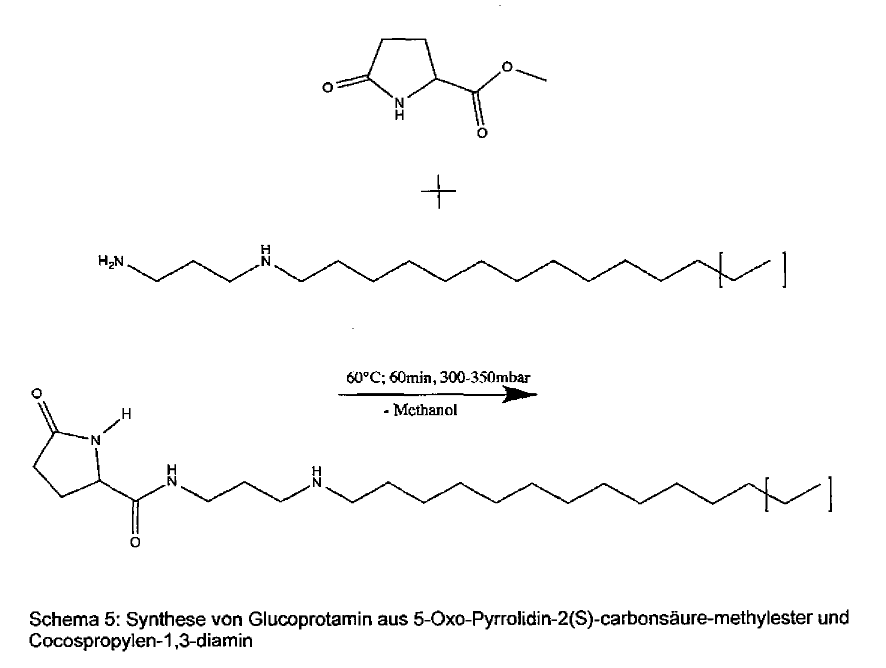

### 3.2 Material und Methode

251 g (1 Mol) Cocospropylen-1,3-diamin (CAS-Nr. 6171-63-7) (70 Mol-% Dodecylpropylendiamin, 30 Mol-% Tetradecylpropylendiamin) wurden im Wasserbad bei 60°C geschmolzen. Anschließend wurden 143,14 g (1 Mol) 5-Oxo-Pyrrolidin-2(S)-carbonsäuremethylester zugegeben und bei reduziertem Druck von 330 mbar im Rotationsverdampfer für 1 Stunde bei 60°C zur Reaktion gebracht. Das bei der Reaktion entstandene Methanol (32g) wurde abdestilliert. Das Umsetzungsprodukt war bei 60°C flüssig-viskos und erstarrte bei Raumtemperatur zu einer beige-gelben wachsartigen Paste. Die Schmelztemperatur des Umsetzungsproduktes beträgt 60-70°C.

### 3.3 Analyse des Syntheseproduktes

Die Analyse des synthetisierten Produktes bestätigt die Substanz Glucoprotamin. Die Ergebnisse der High-Resolution-Massenspektrometrie ergaben eine Massenabweichung der Testsubstanz von nur 0,01 bis 0,04 ppm der theoretischen Massen von [M+H]+ = 253 g/mol (2S)-Pyrrolidin-5-oxo-carbonsäureamid-N-3-(dodecylamino)propyl und [M+H]+ = 286 g/mol (2S)-Pyrrolidin-5-oxo-carbonsäureamid-N-3-(tetradecylamino)propyl.

Eine 1H- und 13C-NMR-Strukturanalyse zeigte die Übereinstimmung mit den theoretisch vorhergesagten Spektren (Scifinder/ChemDraw 13.0).

### 4. Modifizierung der Ausgangssubstanzen

Das neue Herstellungsverfahren ermöglicht aufgrund seiner milden Reaktionsbedingungen den Einsatz von langkettigen und/oder ungesättigten und somit vulnerablen Kohlenstoffketten, ohne dass die Gefahr einer Zersetzung oder Oxidation im Rahmen der Synthesereaktion besteht. Ferner können in dem neuen Herstellungsverfahren bereits vorab "chemisch konfektionierte" Ausgangssubstanzen eingesetzt werden. Auch aufwendige chemische Reaktionen werden ohne Einflussnahme auf die Synthesereaktion möglich.

Die Ausgangssubstanzen 5-Oxo-Pyrrolidin-2-carbonsäure-Derivate und N-substituierte Monoamin-, Diamin- und Fettamid-Derivate sollen mit dem Ziel modifiziert werden, Einfluss zu nehmen auf Wirkstoffkinetik, Wirkstoffdynamik und Wirkstoffleistung der Syntheseprodukte.

### 4.1 Modifizierung der 5-Oxo-Pyrrolidin-2-carbonsäure-Derivate

Die Ausgangssubstanz 5-Oxo-Pyrrolidin-2-carbonsäure kann an den Positionen 3 und 4 des Pyrrolidinrings modifiziert werden. Beispiele sollen unter 4.1.1 und 4.1.2 genannt werden.

### 4.1.1 Anheftung von Gruppen zur Kopplung an Oberflächenmaterialien

Die Anheftung z.B. von geschützten Hydroxyl- oder Sulfhydrylgruppen an die Positionen 3 und/oder 4 des Pyrrolidinrings ermöglichen die Kopplung der Wirksubstanz an Oberflächen verschiedener Materialien. Dies erlaubt die Nutzung der hergestellten Wirkstoffe als Oberflächenbeschichtung.

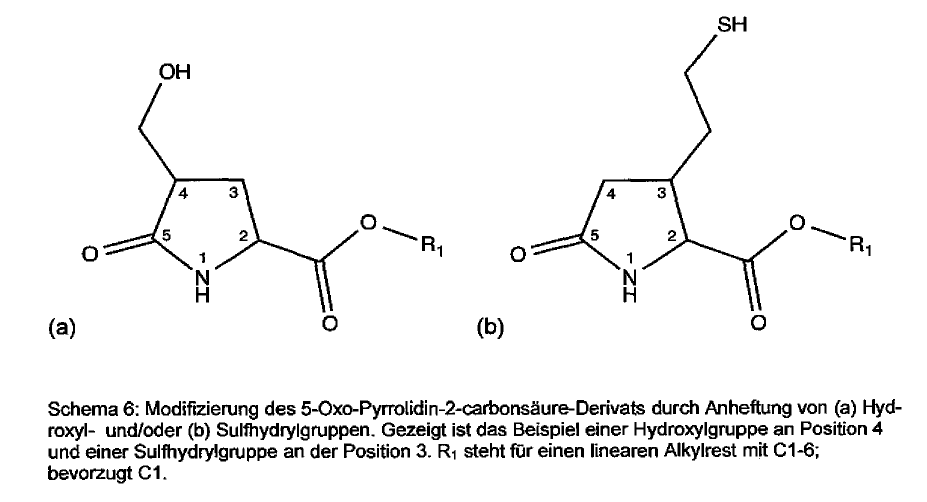

### 4.1.2 Anheftung von Funktionsgruppen, welche eine Modulation oder eine konditionierte Anheftung, Aktivierung oder Inaktivierung des Wirkstoffes ermöglichen

Die Anheftung von z.B. fotoreaktiven Gruppen kann eine konditionierte Modifizierung der Wirksubstanz ermöglichen.

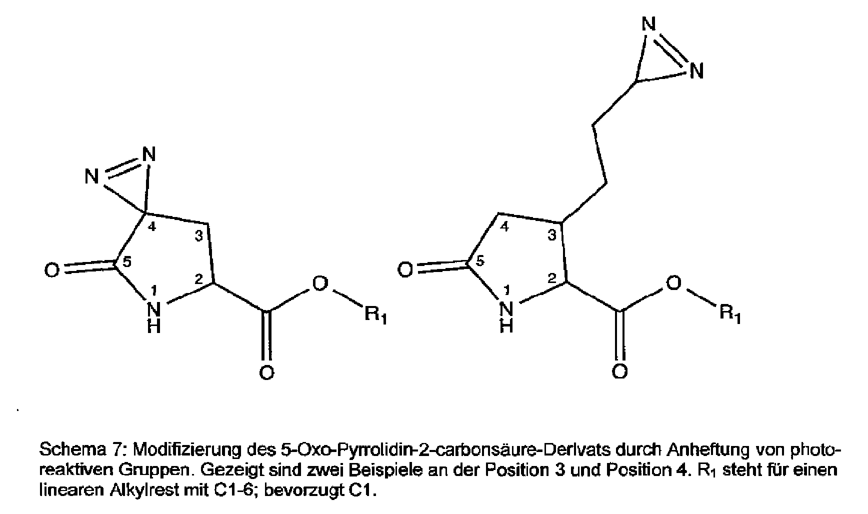

### 4.2 Modifizierung der N-substituierten Monoamin-, Diamin-, Fettamid-Derivate

Das milde erfindungsgemäße Herstellungsverfahren ermöglicht z.B. auch den Einsatz von Alkylresten unterschiedlicher Kettenlängen oder auch im ungesättigten Zustand. Eine Modifizierung der Kettenlänge kann zu einem veränderten Wirkstoffprofil führen.

### (1) Diamine, (2) Monoamine, (3) Fettamide

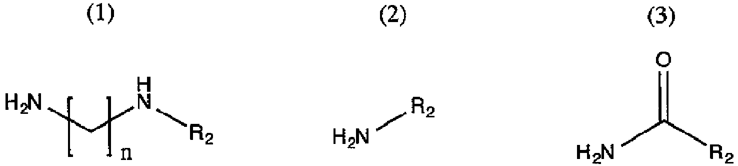

mit R₂ =
- linearer Alkylrest mit einer Kettenlänge von C₂₋₂₂
- einfach und/oder mehrfach ungesättigte Alkylreste
- bei (1) auch einfach und/oder mehrfach ungesättigte Acylreste
- n = 1-6

Schema 8: Darstellung modifizierter Monoamin-, Diamin und Fettamidgruppen.

### 5. Fazit

Die Erfinder stellen zum ersten Mal ein Verfahren zur Herstellung von Pyrrolidinderivaten, wie dem Glucoprotamin, bereit, das im Vergleich zu dem herkömmlichen Verfahren schnell, schonend und energieeffizient ist. Die Herstellung erfolgt mit weniger Nebenreaktionen und erfordert keine aufwendigen Aufreinigungsschritte. In dem erfindungsgemäßen Verfahren lassen sich gezielt Wirkgruppen in die Pyrrolidinderivate einbauen, ohne dass diese durch die Reaktionsbedingungen zerstört werden. Das neue Verfahren ist darüber hinaus besonders kostengünstig.

## Patentansprüche

1. Verfahren zur Herstellung eines Pyrrolidinderivates, das folgende Schritte aufweist:
1) Inkubation der Reaktionspartner
a) 5-Oxo-Pyrrolidin-2-carbonsäure-Derivat mit der Formel I 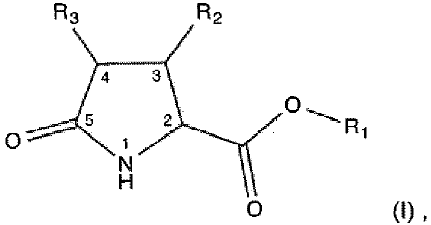 in der
R₁ ein linearer Alkylrest mit C₁-C₆ ist;
R₂ und R₃ jeweils unabhängig voneinander ausgewählt sind aus der Gruppe bestehend aus:
H, XH, 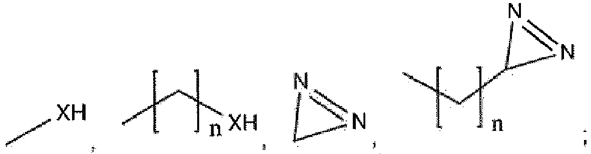 mit jeweils:
n = 0 - 20, und
X = O oder S,
b) einer Verbindung ausgewählt aus der Gruppe bestehend aus:
N-substituiertes Diamin mit der Formel II 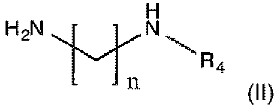 in der R₄ ein linearer Alkyl- oder Acylrest mit C₂ bis C₂₂ ist, und n = 1 - 6 ist; und
N-substituiertes Monoamin mit der Formel III 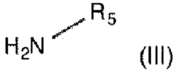 in der R₅ ein linearer Alkyl- oder Acylrest mit C₂ bis C₂₂ ist; und
N-substituiertes Fettamid mit der Formel IV 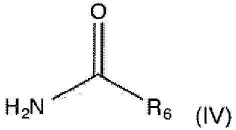 in der R₆ ein linearer Alkylrest mit C₂ bis C₂₄ ist;
unter geeigneten Reaktionsbedingungen, und
2) Isolierung des erhaltenen Pyrrolidinderivates.

2. Verfahren nach Anspruch 1, **dadurch gekennzeichnet, dass** das N-substituierte Diamin und/oder N-substituierte Monoamin und/oder N-substituierte Fettamid an R₄ bzw. R₅ bzw. R₆ einen einfach und/oder mehrfach ungesättigten Alkyl- und/oder Acylrest aufweist.

3. Verfahren nach Anspruch 1 oder 2, **dadurch gekennzeichnet, dass** das 5-Oxo-Pyrrolidin-2-carbonsäure-Derivat ein 5-Oxo-Pyrrolidin-2-carbonsäuremethylester mit der Formel V ist

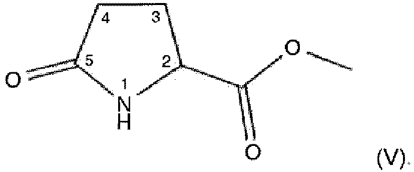

4. Verfahren nach einem der vorherigen Ansprüche, **dadurch gekennzeichnet, dass** das N-substituierte Diamin Cocospropylen-1,3-diamin mit der Formel VI ist:

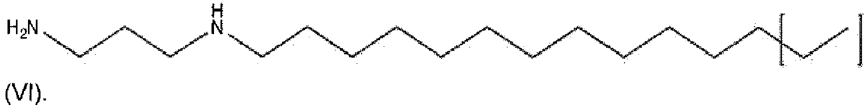

5. Verfahren nach Anspruch 4, **dadurch gekennzeichnet, dass** das Cocospropylen-1,3-diamin 70 Mol-% Dodecylpropylendiamin und 30 Mol-% Tetradecylpropylendiamin aufweist.

6. Verfahren nach einem der vorherigen Ansprüche, **dadurch gekennzeichnet, dass** das Pyrrolidinderivat Glucoprotamin mit der Formel VII ist:

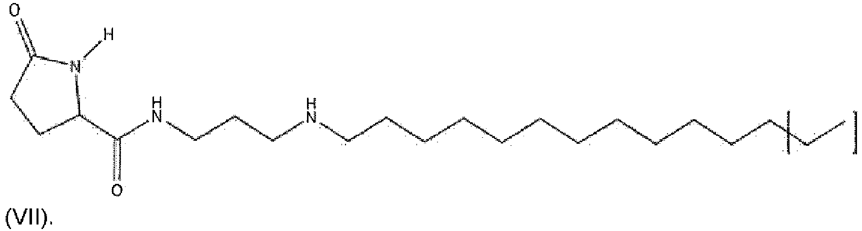

7. Verfahren nach einem der vorherigen Ansprüche, **dadurch gekennzeichnet, dass** die Reaktionsbedingungen eine Inkubationstemperatur von 10°C bis 100°C, vorzugsweise von 30°C bis 90°C, höchst vorzugsweise von 60°C umfassen.

8. Verfahren nach einem der vorherigen Ansprüche, **dadurch gekennzeichnet, dass** die Reaktionsbedingungen eine Inkubationsdauer von 10 min bis 100 min, vorzugsweise von 30 min bis 90 min, höchst vorzugsweise von 60 min umfassen.

9. Verfahren nach einem der vorherigen Ansprüche, **dadurch gekennzeichnet, dass** die Reaktionsbedingungen einen Druck von ≤ 1 bar, vorzugsweise von ≤ 800 mbar, weiter vorzugsweise von ≤ 500 mbar, weiter vorzugsweise von ≤ 400 mbar, und höchst vorzugsweise 330 mbar umfassen.

10. Verfahren nach einem der vorherigen Ansprüche, **dadurch gekennzeichnet, dass** das molare Verhältnis der Reaktionspartner (a) zu (b) gleich 1 zu 1 bis 1 zu 2 beträgt.

11. Verfahren nach einem der vorherigen Ansprüche, **dadurch gekennzeichnet, dass** in Schritt (1) entstandene Reaktionsnebenprodukte entfernt werden.

12. Verfahren nach Anspruch 11, **dadurch gekennzeichnet, dass** das Reaktionsnebenprodukt Methanol umfasst, das durch Destillation entfernt wird.

13. Verwendung eines 5-Oxo-Pyrrolidin-2-carbonsäure-Derivat mit der Formel I 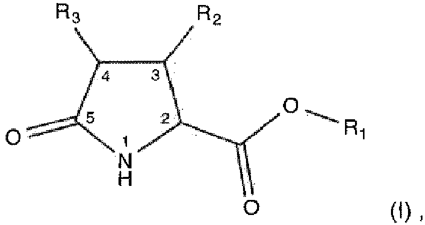 in der
R₁ ein linearer Alkylrest mit C₁-C₆ ist;
R₂ und R₃ jeweils unabhängig voneinander ausgewählt sind aus der Gruppe bestehend aus:
H, XH, 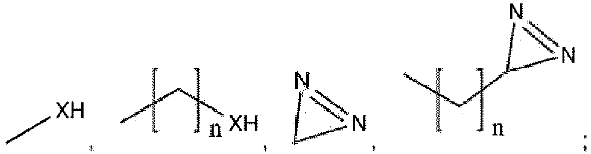 mit jeweils:
n = 0 - 6, und
X = O oder S,
vorzugsweise von
5-Oxo-Pyrrolidin-2-carbonsäuremethylester mit der Formel V 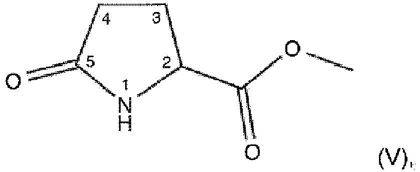 als Ausgangssubstanz zur Herstellung eines Pyrrolidinderivates, vorzugsweise von dem unter die beiden Wirksubstanzen (2S)-Pyrrolidin-5-oxo-carbonsäureamid, N-3-(dodecylamino)propyl und (2S)-Pyrrolidin-5-oxo-carbonsäureamid, N-3-(tetradecylamino)propyl subsumierten Wirkstoff Glucoprotamin.

## Claims

1. A method for the production of a pyrrolidine derivative, which comprises the following steps:
1) Incubation of the reaction partners
a) 5-oxo-pyrrolidine-2-carboxylic acid derivative of the formula I 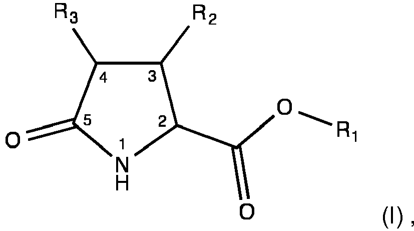 wherein
R₁ is a linear alkyl residue with C₁-C₆;
R₂ and R₃ are each independent from each other selected from the group consisting of:
H, XH, 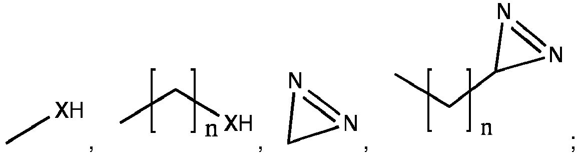 with each:
n = 0 - 20, and
X = O or S,
b) a compound selected from the group consisting of:
N-substituted diamine of the formula II 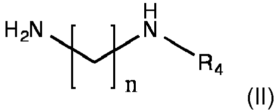 where R₄ is a linear alkyl or acyl residue with C₂ to C₂₂, and n = 1 - 6; and
N-substituted monoamine of the formula III 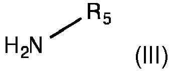 where R₅ is alinear alkyl or acyl residue with C₂ to C₂₂; and
N-substituted fatty amide of the formula IV 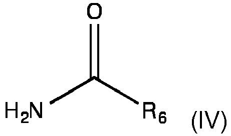 where R₆ is a linear alkyl group with C₂ to C₂₄;
under appropriate reaction conditions, and
2) Isolation of the obtained pyrrolidine derivative.

2. The method of claim 1, **characterized in that** any of the N-substituted diamine and/or N-substituted monoamine or N-substituted fatty amide comprises at R₄ or R₅ or R₆ any of a mono and/or poly unsaturated alkyl or acyl residue.

3. The method of claim 1 or 2, **characterized in that** the 5-oxo-pyrrolidine-2-carboxylic acid derivative is a 5-oxo-pyrrolidine-2-carboxylic acid methyl ester of the formula V

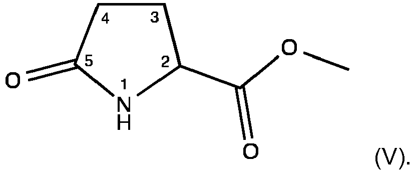

4. The method of any of the preceding claims, **characterized in that** the N-substituted diamine is cocospropylene-1,3-diamine of the formula VI:

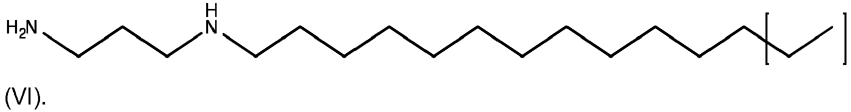

5. The method of claim 4, **characterized in that** the cocospropylene-1,3-diamine comprises 70 mol-% of dodecylpropylenediamide and 30 mol-% of tetradecylpro-pylenediamide.

6. The method of any of the preceding claims, **characterized in that** the pyrrolidine derivative is glucoprotamin of the formula VII:

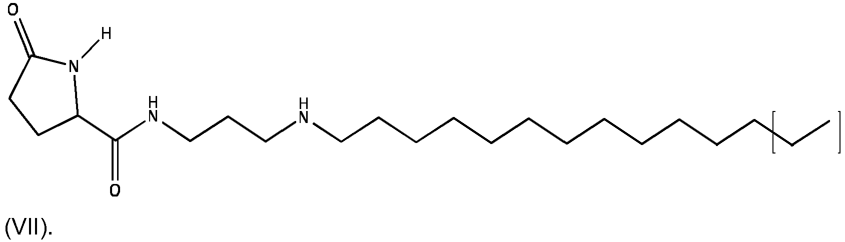

7. The method of any of the preceding claims, **characterized in that** the reaction conditions comprise an incubation temperature of 10° C to 100° C, preferably of 30° C to 90° C, highly preferably of 60° C.

8. The method of any of the preceding claims, **characterized in that** the reaction conditions comprise an incubation time of 10 min. to 100 min., preferably of 30 min. to 90 min, highly preferably of 60 min.

9. The method of any of the preceding claims, **characterized in that** the reaction conditions comprise a pressure of ≤ 1 bar, preferably of ≤ 800 mbar, further preferably of ≤ 500 mbar, further preferably of ≤ 400 mbar, and highly preferably of 330 mbar.

10. The method of any of the preceding claims, **characterized in that** the molar ratio of the reaction partners (a) to (b) is 1 to 1 up to 1 to 2.

11. The method of any of the preceding claims, **characterized in that** reaction side products generated in step (1) are removed.

12. The method of claim 11 **characterized in that** the reaction side product comprises methanol which is removed by distillation.

13. A use of a 5-oxo-pyrrolidine-2-carboxylic acid derivative of the formula I 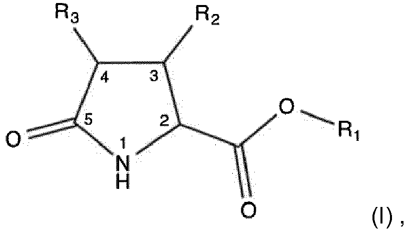 wherein
R₁ is a linear alkyl residue with C₁-C₆;
R₂ and R₃ each independent from each other are selected from the group consisting of
H, XH, 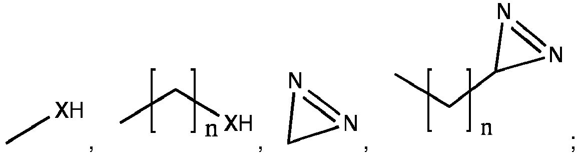 each with:
n = 0 - 6, and
X = O or S,
, preferably of 5-oxo-pyrrolidine-2-carboxylic acid methyl ester of the formula V 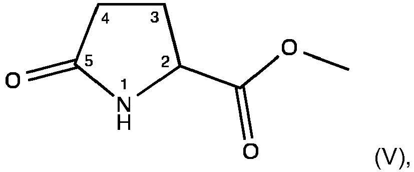 as starting substance for the production of a pyrrolidine derivative, preferably of the two active substances (2S)-pyrrolidine-5-oxo-carboxylic acid amide, N-3-(dodecylamino)propyl and the (2S)-pyrrolidine-5-oxo-carboxylic acid amide, N-3-(tetradecylamino)propyl, which are subsumed under the active agent glucoprotamin.

## Revendications

1. Procédé de production d'un dérivé de pyrrolidine, qui présente les étapes suivantes :
1) l'incubation des partenaires de réaction
a) dérivé d'acide 5-oxo-pyrrolidine-2-carboxylique avec la formule I 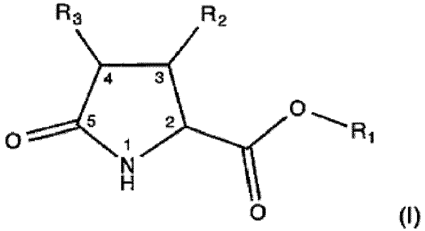 dans laquelle
R₁ est un radical alkyle linéaire ayant 1 à 6 atomes de carbone ;
R₂ et R₃ sont choisis respectivement indépendamment l'un de l'autre parmi le groupe constitué de :
H, XH, 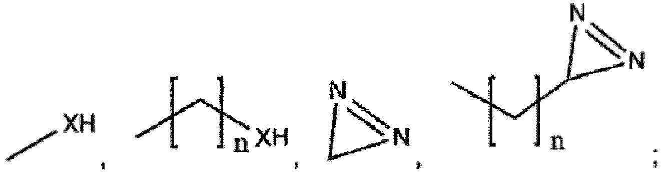 avec respectivement :
n = 0 - 20, et
X = O ou S,
b) un composé choisi parmi le groupe constitué :
d'une diamine N-substituée avec la formule II 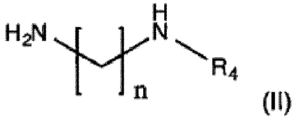 dans laquelle R₄ est un radical alkyle ou acyle linéaire ayant 2 à 22 atomes de carbone et n = 1 - 6 ; et
d'une monoamine N-substituée avec la formule III 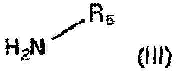 dans laquelle R₅ est un radical alkyle ou acyle linéaire ayant 2 à 22 atomes de carbone ; et
d'un amide gras N-substitué avec la formule IV 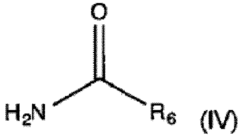 dans laquelle R₆ est un radical alkyle linéaire ayant 2 à 24 atomes de carbone ;
dans des conditions de réaction adaptées, et
2) l'isolement du dérivé de pyrrolidine obtenu.

2. Procédé selon la revendication 1, **caractérisé en ce que** la diamine N-substituée et/ou la monoamine N-substituée et/ou l'acide gras N-substitué présentent au niveau de R₄ ou R₅ ou R₆ un radical alkyle et/ou acyle mono-insaturé et/ou polyinsaturé.

3. Procédé selon la revendication 1 ou 2, **caractérisé en ce que** le dérivé d'acide 5-oxo-pyrrolidine-2-carboxylique est un ester méthylique d'acide 5-oxo-pyrrolidine-2-carboxylique avec la formule V

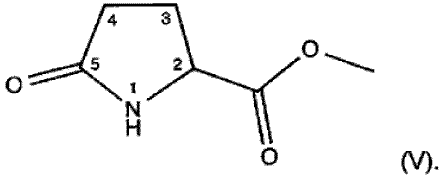

4. Procédé selon l'une quelconque des revendications précédentes, **caractérisé en ce que** la diamine N-substituée est une cocospropylène-1,3-diamine avec la formule VI :

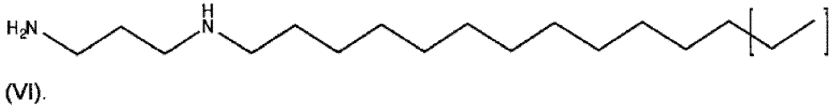

5. Procédé selon la revendication 4, **caractérisé en ce que** la cocospropylène-1,3-diamine présente 70 % en mol. de dodécylpropylènediamine et 30 % en mol. de tétradécylpropylènediamine.

6. Procédé selon l'une quelconque des revendications précédentes, **caractérisé en ce que** le dérivé de pyrrolidine est une glucoprotamine de la formule VII :

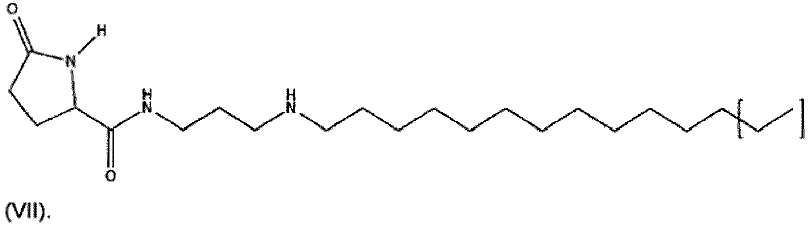

7. Procédé selon l'une quelconque des revendications précédentes, **caractérisé en ce que** les conditions de réaction comprennent une température d'incubation de 10 °C à 100 °C, de préférence de 30 °C à 90 °C, au maximum de préférence de 60 °C.

8. Procédé selon l'une quelconque des revendications précédentes, **caractérisé en ce que** les conditions de réaction comprennent une durée d'incubation de 10 min à 100 min, de préférence de 30 min à 90 min, au maximum de préférence de 60 min.

9. Procédé selon l'une quelconque des revendications précédentes, **caractérisé en ce que** les conditions de réaction comprennent une pression ≤ 1 bar, de préférence ≤ 800 mbar, de préférence encore ≤ 500 mbar, de préférence encore ≤ 400 mbar, et au maximum de préférence de 330 mbar.

10. Procédé selon l'une quelconque des revendications précédentes, **caractérisé en ce que** le rapport molaire entre les partenaires de réaction (a) et (b) est égal à 1:1 à 1:2.

11. Procédé selon l'une quelconque des revendications précédentes, **caractérisé en ce que** des produits secondaires de réaction apparus à l'étape (1) sont éliminés.

12. Procédé selon la revendication 11, **caractérisé en ce que** le produit secondaire de réaction comprend du méthanol, qui est supprimé par distillation.

13. Utilisation d'un dérivé d'acide 5-oxo-pyrrolidine-2-carboxilique avec la formule I 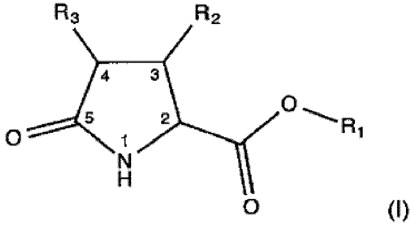 dans laquelle
R₁ est un radical alkyle linéaire ayant 1 à 6 atomes de carbone ;
R₂ et R₃ sont choisis respectivement indépendamment l'un de l'autre parmi le groupe constitué de :
H, XH, 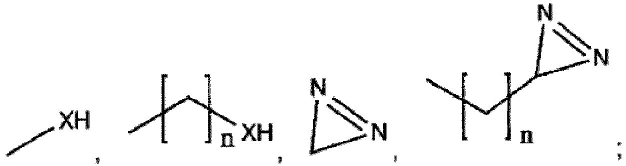 avec respectivement
n = 0 - 6, et
X = O ou S,
de préférence
d'ester méthylique d'acide 5-oxo-pyrrolidine-2-carboxilique avec la formule V 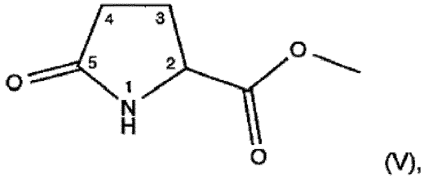 en tant que substance de départ servant à produire un dérivé de pyrrolidine, de préférence du principe actif de la glucoprotamine rattachée à l'une des deux substances actives d'amide d'acide (2S)-pyrrolidine-5-oxo-carboxylique, N-3-(dodécylamino)propyle et d'amide d'acide (2S)-pyrrolidine-5-oxo-carboxylique, N-3-(tétradécylamino)propyle.
